# EUROPEAN PATENT APPLICATION

(11) **EP 3 473 162 A1**
(43) Date of publication of application: **24.04.2019**
(21) Application number: 17196875.3
(22) Date of filing: 17.10.2017
(51) Int. Cl.: A61B 5/00, A61B 5/11, A61B 5/22

(54) **WRIST-WORN GRIP MEASUREMENT DEVICE**

(71) Applicant: Koninklijke Philips N.V., 5656 AE Eindhoven (NL)
(72) Inventor: JOHNSON, Mark K., 5656 AE Eindhoven (NL); AARTS, Ronaldus Maria, 5656 AE Eindhoven (NL); HENDRIKS, Cornelis Petrus, 5656 AE Eindhoven (NL); VAN EE, Raymond, 5656 AE Eindhoven (NL)
(74) Representative: de Haan, Poul Erik

(57) **Abstract**

A wrist-worn grip measurement device comprises a wrist-mountable band which carries one or more force sensing means configured to measure a grip force applied to the band between at least two application points, the points being on surfaces of the device exposed during wearing to permit application of the force by an alternate hand, and having a controller configured to process outputs of the force sensing means and generate a signal indicative of a grip force applied between said points. The grip force may be a gripping or pinching force.

## Description

### FIELD OF THE INVENTION

This invention relates to a wrist-worn device for measuring grip strength, and in particular for measuring grip or pinch strength.

### BACKGROUND OF THE INVENTION

Measurement of grip or pinch strength is known to reflect the status of some chronic diseases such as arthritis and motor neuron disease and can usefully provide indication of any hand-based injuries such as strains and sprains, including both detection and the monitoring of rehabilitation. Such functions are particularly useful for elderly people who are more prone to chronic diseases and hand or wrist based injuries. Measurement of grip of pinch strength is also of use in other (non-clinical) contexts, such as in recreational strength training.

Wearable health devices are growing in use and popularity and provide convenient means for monitoring hand or wrist parameters.

US 2012/0277014 describes a wrist strap including a pressure sensor on an inside of the band responsive to wrist muscle expansion, to thereby provide a measure of a gripping pressure being applied on a golf club held by the user's hand. The more tightly the club is gripped, the greater the wrist muscle expansion, and the stronger the pressure signal at the pressure sensor.

However, this device does not provide an objective measure of the force of a user's grip or pinch. The device is only able to give a relative indication of comparative grip pressure applied to a golf club on different occasions; expansion of a wrist muscle does not give a quantitative measure of wrist muscle strength.

The device is also unable to provide a measure of pinch strength.

There is sought an improved wrist-worn device capable of facilitating measure of a force of a user's grip or pinch.

### SUMMARY OF THE INVENTION

The invention is defined by the claims.

According to examples in accordance with an aspect of the invention, there is provided a wrist-worn grip measurement device comprising: a band for fitting on a wrist; a force sensing means carried by the band for sensing a grip force applied to the device between at least two points, said points being on surfaces exposed during wearing to permit application of the force by a user's alternate hand; and a controller adapted to generate a signal indicative of the grip force applied between said points based on an output of the force sensing means, and to generate an output based on said signal.

The invention hence provides a wrist-wearable band having force sensing means arranged to permit access by a user's alternate hand (i.e. alternate to a hand on which the device is worn). The force sensing means is configured to sense and generate a signal indicative of a grip force applied by said alternate hand to the device between at least two points. Hence in use, the user grips, e.g. squeezing or pinching the device between two points, the points having mechanical communication with the force sensing means to permit measurement of the force applied between those points. The points are on externally accessible surfaces of the device, i.e. surfaces not in contact with or facing a user's wrist. Since the force is measured between two force application points, an objective, quantitative measure of an applied grip force can be obtained.

The controller may generate a signal directly indicative of a grip force or indirectly indicative of a grip force. The controller may process the output of the force sensing means to generate a quantitative measure of an applied grip force. The controller may alternatively output a signal correlated with applied force, e.g. a strain signal of a strain gauge, from which a measure or determination of grip force can be obtained.

The controller may comprise or include a processor for processing output signals of the force sensing means to generate said signal indicative of a grip force.

The signal indicative of the grip force generated by the controller may be an analog signal, or otherwise a signal representative of grip force as measured as a continuous variable or physical quantity.

The output generated by the controller based on the generated signal may be directly representative of the generated signal. The controller may output the generated signal as the generated output. i.e. as a signal output.

By 'grip force' is meant any compressive force applied between two or more inwardly pressing points of the hand. This could be a grip force applied between two fingers, i.e. a pinching force, or could be a grip force applied by the hand more broadly, i.e. a grasping grip force.

The force sensing means may generally be any means capable of providing an indication or measure of the force of a gripping force applied between the at least two points. The force sensing means may by way of example be a pressure sensing element or a force sensor element, either of which provide a measure indicative of applied force.

In accordance with one or more embodiments, the band may form a closed loop shape when worn. The band may be configurable to form a closed loop when worn to enclose the user's wrist. The band may have fastening means to fasten it in a closed loop form around the user's wrist. A closed loop form lends the band greater structural rigidity: applied gripping forces are distributed circumferentially around the loop, rather than being directed exclusively inwardly toward the user's wrist. Where the band forms only a partial loop, inward application of force leads to bending of wing portions of the loop inward onto the user's wrist, which causes discomfort and impedes functionality. Where a closed loop is provided the loop forces are circumferentially distributed, the structure of the band providing resistance to radial deformation.

In accordance with any embodiment, there may be provided a plurality of force sensing means.

According to one or more embodiments the force sensing means may comprise a strain gauge means having a deformable element, the strain gauge means being adapted to provide a measure of a strain of the deformable element. The deformable element may have a flexibility which is greater than a flexibility of surrounding portions of the band. This may allow that a majority of force applied in the area of the strain gauge is absorbed by the deformable element, preventing undue deformation of surrounding areas and so preserving structural integrity or rigidity.

The force sensing means may be integral to the band. By this is meant the force sensing means may be integrally comprised in the band, e.g. formed within the band, embedded in the band, enveloped within the band, or incorporated within the body of the band.

In accordance with a first set of embodiments, the band may include a resilient circumferential (sensing) portion of greater relative resilience than a remaining circumferential (strap) portion, the resilient circumferential portion being flexible inwardly of the band upon exertion of a gripping force by the user, and the resilient circumferential portion incorporating strain gauge means, for measuring of a strain of the resilient circumferential portion upon said flexing.

This set of embodiments is based on constructing the band with a resilient circumferential portion configured to flex radially inwardly upon application of a gripping force by the user's alternate hand. However, since the resilient portion has greater relative resilience than surrounding portions, the resilient portion absorbs a majority of a gripping force applied across a region occupied by the resilient portion. Hence, only minimal force is transferred across the less resilient remainder of the band, limiting the extent to which this part of the band is deformed inwardly by the force which could lead to pressing of the band onto the user's wrist, thereby causing discomfort and impeding functionality. This is an example for a device with a soft strap.

By greater relative resilience may be meant lower relative elasticity (than the surrounding circumferential portion). This may for instance be measured in N/m.

By greater relative resilience may be meant greater flexural rigidity.

By resilient may be meant radially resilient; resilient to flexing in a generally radially inward direction. Resilience is in general a measure of the ability of a material to resist strain. So by greater resilience may be meant lower flexibility in directions generally inward of the band.

In accordance with preferred examples, the resilient circumferential portion may occupy less than half of a total circumference of the band.

By reason of this, assuming the resilient portion is orientated in a dorsal position when worn, flexing of this portion substantially avoids bending of the band onto the user's wrist bones. A typical human wrist may be said to describe a roughly elliptical type cross-section, with the sides of the wrist bones aligned at vertices of the ellipse shape, i.e. approximately 180 degrees apart. The resilient portion, which occupies less than 180 degrees of the loop, hence does not reach as far as the sides of the bones. Flexing of the resilient portion therefore does not press this portion onto the sides of the bones. The less resilient portion is also not pressed onto the bone, by virtue of the fact that the resilient portion absorbs the majority of the applied force. This hence minimises discomfort and any possible impeding of functionality.

The resilient circumferential portion may be formed by an arcuate elastic member.

The surrounding circumferential portion refers to portions of the circumference either side of the resilient portion to which the resilient portion is immediately joined or connected. The surrounding portions may extend to form the remainder of the band. The surrounding less resilient portions may be formed of a relatively compliant material such as silicone or fabric for instance.

In examples, the resilient circumferential portion may be formed by a resilient elastic member incorporated integrally within a surrounding compliant band.

In another set of examples, the band includes a set of rigid links; and one or more force sensing means are provided between adjacent rigid links. In this case, the strap itself provides a shield between the external grip and the wrist, so that the force sensing is by a more elastic (less resilient) member. This example is for example for a metal articulated link strap.

Thus, various arrangements are possible to enable grip force measurement without the applied force transferring to the underlying wrist.

In a further set of embodiments, the force sensing means may comprise at least one pair of force sensor elements carried by the band, and circumferentially spaced to permit gripping of the band between the force sensor elements to permit measurement of the user's grip. The force sensor elements are accessible to a user during wearing of the band to permit measurement of a grip force applied to the band by an alternate hand. Each one of the pair of elements provides one of the force application points and a grip force is applied by the user between the two elements. Outputs of the pair may be processed by the controller together and/or separately to provide a measure or indication of a grip force applied to the elements.

The force sensor elements may be incorporated within the body of the band or may be external elements mounted on the band.

In examples, the at least one pair of force sensor elements may be circumferentially separated by greater than one quarter of a circumference of the band; and/or the at least one pair of force sensor elements may be arranged diametrically opposite to one another.

The at least one pair of force sensor elements may each be supported by a flexible backing layer to prevent buckling and to achieve a more evenly distributed pressure.

In examples, the band may carry a plurality of said pairs of force sensor elements. The plurality of pairs may be provided in the form of a sensor patch embodying e.g. an annular array of force sensing elements. In this way it is better assured that there is at least one sensing element aligned with gripping points of the user's hand when they grip the device.

In accordance with one or more embodiments, the force sensing means may comprise a pinch sensing element which includes a layer element having edges accessible to a user and being bendable by in-plane compression, and incorporating strain gauge means to measure the bending and thereby provide a measure of a force of said in-plane compression.

The pinch sensing element may be integrated in the body of the band or may be provided as an external element mounted on or coupled to the band.

The wrist-worn device may comprise a fastening element to permit fastening of two ends of the band around the user's wrist, and wherein the force sensing means is comprised by the fastening means. The fastening means may by way of non-limiting example include a clasp or clamp or buckle.

The fastening means may in examples integrally comprise the pinch sensing element described above.

It is anticipated that embodiments may combine multiple force sensing means in one device. For instance a device may comprise both a grip sensing means as described in any example above, and a separate dedicated pinch sensing means in accordance with examples described above.

Any described embodiment may be combined with any other embodiment, such that in variations multiple of the example grip measurement and/or pinch measurement means are included in the same device.

It is advantageous that grip measurements are taken at regular intervals throughout the day or at particular times of the day, for instance coinciding with particular points of the patient's circadian cycle.

The controller may hence in accordance with one or more embodiments be adapted to produce sensory signals in accordance with a stored measurement schedule to prompt a user to perform hand strength measurements at one or more scheduled times.

The controller may additionally and/or alternatively be adapted to log the generated signals indicative of a grip force in order to obtain a record of grip measurements at different times of day and over different days, weeks or months.

The logging may comprise storing the indications on a local memory, and/or may comprise communicating the indications as output information to a remote data store, e.g. a memory on a remote server.

In accordance with an aspect of the invention, there is provided a grip strength measurement method based on use of a wrist-worn grip measurement device comprising: a band for fitting on a wrist, and a force sensing means carried by the band for sensing a grip force applied to the device between at least two points, said points being on surfaces exposed during wearing to permit application of the force by a user's alternate hand; the method comprising: receiving one or more output signals from the force sensing means, generating a signal indicative of a grip force applied between said at least two points based on said one or more output signals and generating an output based on said signal indicative of a grip force.

In accordance with one or more embodiments, the method may further comprise generating one or more sensory outputs in accordance with a stored measurement schedule to prompt a user to perform hand strength measurements at one or more scheduled times. The sensory output may be an auditory, visual or haptic signal for instance.

### BRIEF DESCRIPTION OF THE DRAWINGS

Examples of the invention will now be described in detail with reference to the accompanying drawings, in which:
Fig. 1 shows an example wrist-worn grip measurement device in accordance with one or more embodiments of the invention;
Fig. 2 shows a further example wrist-worn grip measurement device in accordance with one or more embodiments of the invention;
Fig. 3 shows a top-down view of an example pinch sensing element for use in devices in accordance with one or more embodiments of the invention;
Fig. 4 shows a sectional view of the example pinch sensing element of Fig. 3; and
Fig. 5 shows an example wrist-worn grip measurement device comprising a pinch sensing element.

### DETAILED DESCRIPTION OF THE EMBODIMENTS

The invention provides a wrist-worn grip measurement device comprising a wrist-mountable band which carries one or more force sensing means being configured to measure a grip force applied to the band between at least two application points, the points being on surfaces of the device exposed during wearing to permit application of the force by an alternate hand, and having a controller configured to process outputs of the force sensing means and generate a signal indicative of an applied grip force. The grip force may be a gripping or pinching force.

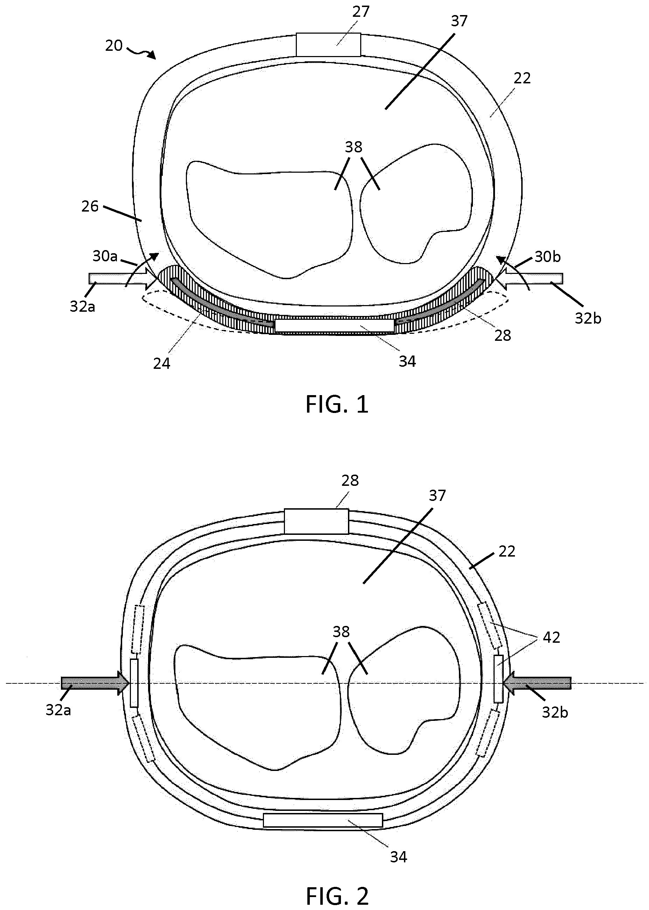

Fig. 1 shows a first example wrist-worn grip measurement device 20 in accordance with one or more embodiments of the invention. The device comprises a band 22 having a clasp 27 which permits fastening of two ends of the band together about a user's wrist 37 to form a closed loop shape which completely encloses a user's wrist when worn. The user's wrist is indicated generally at 37, with wrist bones indicated by 38. The band comprises a first circumferential portion 24 for force sensing and a second circumferential portion 26 generally forming the connection strap. The first portion is resilient but sufficiently flexible able to permit radial inward flexing of the band. It has greater resilience than the second portion. The first circumferential portion forms a first part of the band and the second circumferential portion forms the remainder of the band. The second (strap) portion may surround the first in that the first (sensing) portion 24 may be embedded in the second portion 26. The first (sensing) portion may be attached to a surface or to the ends of the second (strap) portion. The second portion 26 defines a soft band to provide good comfort and fit, whereas the first potion 24 is for measurement of the grip force.

By 'greater resilience' may be meant in general of lower relative elasticity or flexibility.

Greater resilience may mean of greater resilience with regard at least to straining in a generally radially inward direction, as indicated by arrows 30a and 30b, i.e .offering greater resistance to such inward flexing, i.e. being less flexible with regards such force.

The first portion will be referred to as the 'resilient circumferential portion'.

The resilient circumferential portion 24 comprises an arcuate strain gauge element 28 extending circumferentially through a body of the resilient portion. The arcuate strain gauge element provides a force sensing means. The strain gauge element is configured to provide measurement at least of radially inward straining of the resilient circumferential portion 24 in the direction of arrows 30a and 30b. In the example of Fig. 1, the strain gauge element is integral of the band, and in particular integral of the resilient circumferential portion.

The resilient circumferential portion preferably extends to less than one half of the total circumference of the band 22, as illustrated in the example of Fig. 1. This allows that applied gripping force does not lead to bending of the band inwards in a manner that leads to commensurate gripping of the wrist bones 38 between deformed wings of the band.

In use, the band is fitted around a user's wrist 37, for instance by unclasping the two ends of the band 22 to wrap or mount the band onto the wrist, and then fastening the ends together around the band using the clasp 27. Once fitted, grip measurement is performed by gasping or gripping the outside of the band with an alternate hand to the one to which the band is fitted. The user preferably orients their alternate hand when so gripping such that the gripping force is applied inwardly between the two application points 32a, 32b, located at the ends of the flexible circumferential portion. This ensures that the majority of the applied force is exerted across the resilient portion, so that an obtained measure of applied force can be as accurate as possible.

When a gripping force is so applied by a user, the force is elastically absorbed by the resilient circumferential portion 24 of the band, causing it to flex inwardly as indicated by arrows 30a, 30b. The extent of the flexing is measured or detected by the strain gauge element 28 embodied within the resilient circumferential portion. The strain gauge element is communicatively coupled to a controller 34. Based on signal outputs of the strain gauge element, the controller generates a signal indicative of a grip force applied by the user to the band.

This may in examples comprise processing the output of the strain gauge element in accordance with an equation or algorithm to arrive at a grip force measurement, or may involve use for instance of a locally or remotely stored lookup table to determine the force measurement based on the strain gauge output. In examples, the grip force indication may be calculated by the controller. It may be calculated based on a known elasticity or flexibility of the resilient circumferential portion.

In the example of Fig. 1, the resilient circumferential portion 24 occupies less than one half of the total circumference of the band 22. Upon application of the gripping force by a user, due to its greater relative resilience, the only substantial flexing occurs across the resilient portion of the band, and leads to flexing of this portion inwardly, as indicated by arrows 30a, 30b. The surrounding portion 26 remains substantially undeformed. It will be appreciated, that such an arrangement protects the wrist from direct transfer of the gripping force onto the bone 38, which might otherwise occur were the resilient deformable portion to extend across a complete half of the band or more. In the latter case, parts of the band wrapped around the bone would be deformable and upon application of gripping force would lead to inwards pressing on the bone.

Provision of a band shaped to form a complete loop when worn is preferred. Where the band forms only a partial loop around the wrist, in order to avoid natural inward flexing of ends of the loop together upon application of a gripping force, it is necessary to make the all parts of the loop of very rigid (non-flexible) material. However, such very rigid material is both uncomfortable for the user and also, more significantly, interferes with the force measuring functionality since it prevents the straining of the band which permits the applied force to be measured.

By providing a completely closed loop with an elastic arcuate portion, this difficulty is solved since the closed form provides a natural self-resistance to inward deformation of the band (since the applied force is diverted circumferentially around the band, rather than radially inwardly), and the elastic portion allows the limited degree of flexing required to achieve an accurate measurement of applied grip force.

The size of the band should be configured for fitting around a user's wrist. The band 22, and the resilient portion 24 of it, should be sized such that when the band is worn, the resilient portion extends substantially above the wrist bones (as shown in Fig 1), but not to the sides of the bones (so as to avoid the problems discussed above).

A size of the band (i.e. diameter and/or circumference) may in examples be based on an average size of the wrist of an adult person (e.g. a "one size fits all" design). Alternatively, the band may be provided in different sizes for different typical wrist sizes, e.g. small, medium, large. By way of example only, the band may for instance have a circumference between 10 cm and 25 cm. The band might have an adjustable size, the adjustability being configured such as not to interfere with the circumferential rigidity of the band.

The elasticity and resilience of the resilient circumferential portion 24 may be configured such that a maximum grip force of a user leads to a deformation of the resilient portion which is smaller than the size difference of the unloaded resilient portion and the bones 38, such that the grip force is always transmitted to the resilient portion 24 and not blocked by the bones. By way of simple example, as a first order estimate, the required elasticity, based on a maximum grip force of 50 kg and maximum allowed deformation of 5 mm, is approximately 10⁵ N/m.

In further examples, and by way of non-limiting illustration only, elasticity of the resilient circumferential portion may be between 5 x 10⁴ N/m and 5 x 10⁶ N/m, or more preferably between 1 x 10⁵ and 1 x 10⁶ N/m.

The curvature of the resilient circumferential portion 24 is preferably such that it does not wrap around the bones 38, in order not to impair the fit and comfort of the device, or the functionality (by impeding natural flexing and thereby interfering in force measurement).

In examples, the resilient circumferential portion 24 may be provided by an arcuate elastic member mounted relative to the less resilient portion 26 so as to form the resilient portion.

Although a strain gauge element is shown embedded within a body of the resilient circumferential portion 24 of the band, this is not essential. In other examples, the strain gauge element may be otherwise carried or coupled to the resilient portion, e.g. coupled to a surface of the resilient portion.

Although the band 22 is shown having a clasp 27, other fastening means may alternatively be employed to fasten the band ends together when worn, as will be apparent to the skilled person. Further, a band having separable ends and fastening means to secure the ends about a user's wrist is not essential and instead any other means may be provided for permitting mounting to and removal from the wrist of the band.

The surrounding less resilient portion 26 of the band 22 may in examples be formed of a compliant (soft) material, for example a fabric or silicone rubber or a gel, or may in examples be formed of plastic.

The resilient portion 24 may in particular examples be formed of plastic. However, other suitable materials will be apparent to skilled person, and the invention is not limited to use of any particular material for any particular part.

In examples, the resilient portion may be moulded within a surrounding compliant band structure or may be co-operatively received within an internal holding cavity of a surrounding band, or may be integral to such a band, or be may enveloped by such a band. In other examples, it may be joined to the less resilient portions 26 of the band.

The example above makes use of a soft strap and a more resilient part for force measurement.

An alternative arrangement is to make use of a hard strap comprising a set of articulated hard links. These have the structure of a conventional sectioned watch strap. This arrangement allows a hard grip to be applied without the gripping force being applied to the underlying wrist, because the links of the metal strap lock against each other. A strain gauge may then be applied between the articulated links to measure the grip force.

Another alternative arrangement is again a hard strap comprising a set of articulated hard element links but with a more resilient element between an adjacent pair of hard links. This resilient element deforms in response to an applied gripping force, and a strain gauge, for example on the inner surface, may then measure the strain. Of course multiple strain gauges may be applied at different locations around the strap.

Thus, in this example, the band includes a set of rigid links and one or more strain gauge means provided between adjacent rigid links.

The common purpose of all examples above is to enable a grip to be applied outside the strap, for the grip force to be measured, but for the transmission of that grip force to the underlying wrist to be inhibited. In some examples, the strap is soft i.e. elastic so that the force measurement device itself needs to absorb the applied grip force. Thus, it then comprise a more resilient member attached to or embedded in the soft strap. In other examples, the strap is rigid/resilient, so it can absorb the grip force and the force measurement device is then more elastic/less resilient.

In accordance with a further set of embodiments, the device may comprise one or more pairs of force sensor elements being carried by the band to provide force sensing means, the force sensor elements being circumferentially spaced to permit gripping of the band between the force sensor elements and measurement of the user's grip between the elements.

Fig. 2 shows an example of such an embodiment.

As in the previous example, the band 22 is mountable on a user wrist by means of clasp 27 and forms a closed loop when worn. The band carries on opposing sides of the band 22 respective sets of three force sensor elements 42. The two sets of force sensor elements together form three pairs of diametrically opposed force sensor elements. In the example of Fig. 2, the elements are integrally comprised in the body of the band 22. However, in other examples the elements may be external of the band, for instance carried by or mounted on a surface of the band. Although three pairs of force sensor elements are shown in the example of Fig. 2, this is purely for illustration, other numbers of pairs of elements may alternatively be provided.

In use, upon application of a gripping force by a user's alternate hand between e.g. application points 32a, 32b, the force is applied to at least one force sensor element 42 on each side of the band 22. As such the force is applied to the band between at least one pair of circumferentially spaced (in this case diametrically opposed) force sensor elements. The force sensor elements are each communicatively coupled to a controller 34 which generates, based on outputs of the force sensor elements, an indication of the applied grip force between them.

By providing multiple pairs of force sensor elements 42, circumferentially spaced from one another (as in Fig. 2), it is better ensured that a grip force applied by a user aligns with at least one pair of sensor elements. In further examples, sensor elements may be distributed around the entire circumference of the band 22, for instance in an annular array formation. This would better ensure that an applied grip force is measured regardless of where on the band the user applies their grip. The controller may comprise or make use of an algorithm for determining the particular pair of sensor elements between which the user is mainly applying a force. This could be determined for instance by identifying the opposing sensor elements for which the greatest force or pressure is registered. The controller may then calculate or otherwise determine an applied grip force based on this pair of sensor elements in isolation of other pairs. This ensures that force measurements are not double-counted.

The force sensor elements may be or comprise pressure sensors, and may in non-limiting examples comprise electrical resistors configured to measure force or pressure. Any variety of force or pressure sensor may however be used. The force sensing element may provide an output either directly or indirectly indicative of force. The force sensing element may be resistive or capacitive. The force sensing element may include a strain gauge.

Optionally the force sensing elements may be supported with a backing layer, e.g. a flexible backing layer, to prevent buckling and to achieve a more evenly distributed pressure.

In accordance with at least one set of embodiments, there is provided a wrist-worn device adapted to measure a grip force applied between two fingers of a user, i.e. a pinching force.

In accordance with these embodiments, the device 20 may comprise a pinch sensing element having force sensing means configured to provide measurement of a pinch force applied to the element. The pinch sensing element is arranged having points or surfaces accessible to a user during wearing of the device and between which a pinching force can be applied, there being force sensing means provided between those points to measure the pinching force.

The pinch sensing element may be provided integral to the band 22 or may be carried by the band, e.g. coupled or mounted to an external surface.

In examples, a force sensing means may be comprised by the clasp 27. The clasp provides a convenient, accessible housing for the force sensing means, permitting easy access for force application points for a user.

The clasp 27 may in particular comprise a pinch sensing element, and preferably integrally comprises the pinch sensing element. The clasp is a convenient size for carrying the pinch sensing element, since it provides force application points which are relatively proximal to one another which is suited to applying a pinching action, as opposed to a gasping action.

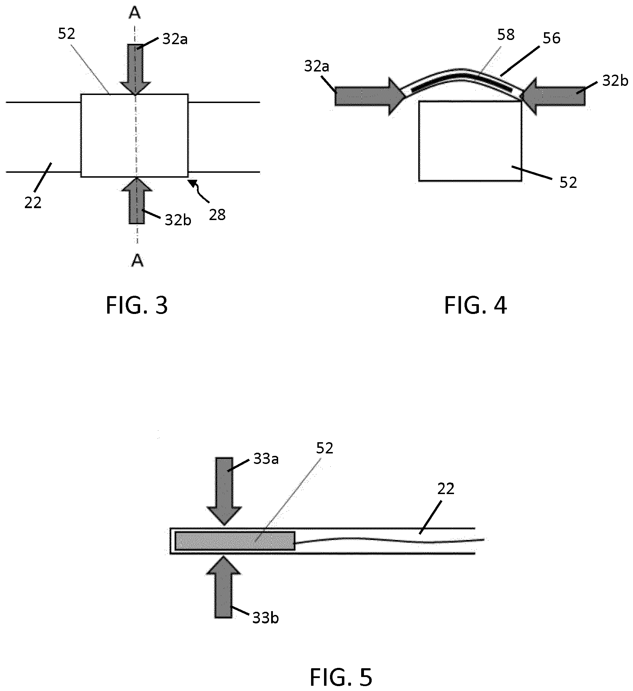

An example pinch sensing element for use in a wrist-worn device according to the invention is illustrated in Figs. 3 and 4. The figures show an example pinch sensing element 52 incorporated within a clasp 27 of the device for fastening two ends of a band 22 of the device together around a user's wrist. Fig. 3 shows a top-down view of the clasp comprising the pinch sensing element. Fig. 4 shows a sectional view through the pinch sensing element across plane A.

The pinch sensing element comprises an upper layer element 56 having edges accessible to a user and being bendable by in-plane compression, and incorporating a strain gauge element 58 to measure the bending and thereby provide a measure of a force of said in-plane compression.

In use, a user may apply a pinching force to the element between force application points 32a and 32b, causing bending of the layer element 56 of an amount commensurate with the applied force. Through measuring the resultant strain, the strain gauge element 58 provides indication of the force applied.

A controller 34 (not shown in Figs. 3 and 4) generates a signal indicative of an applied force based on outputs from the strain gauge element 58.

In the example illustrated in Figs. 3 and 4, the layer element 56 forms a deformable cover plate, extending over the clasp 27. The cover plate is hence mounted to the clasp but not integral to it. In further examples, the plate may be integral to the clasp.

In further examples, the pinching element may take a different form, for instance a pair of pressure sensors mounted at either side of the clasp, either external to the clasp or integral to the clasp.

In accordance with any embodiment, further to a primary grip sensing means, there may be provided a secondary grip sensing means comprised by the band 22 permitting measurement of pinch force applied by a user between opposing points on an upper and lower surface of the band.

An example is illustrated in Fig. 5 which shows a pinch sensing element 52 incorporated in the band 22 of a wearable device. The pinch sensing element facilitates measurement of a pinching force through application of force applied between its upper and lower surfaces. The pinch sensing element is arranged within the band such that a pinching force can be applied across the element via force application points 33a, 33b on upper and lower surfaces of the band. In this case, at least the region of the band 22 proximal the pinch sensing element 52 needs to be structured (e.g. materially structured) to permit transfer of the force from the application points to the pinch sensing element. For example, the region of the band proximal the pinch sensing element may be sufficiently flexible or deformable to permit transfer of the force.

The pinch sensing element 52 may be positioned or sized within the band 22, such that only a thin layer of material extends between the upper and lower surfaces of the element and upper and lower surfaces of the band 22, e.g. a layer less than 20% of the thickness of the band may extend above each of the upper and lower surfaces or less than 15% or less than 10%.

Incorporating a pinch sensing element 52 in the band 22 of the device may in accordance with one or more examples permit measuring of a pinch strength during removal and application of the device from a user's wrist. For example, when removing the band 22, the user may pinch between two points of the band known to align with upper and lower surfaces of the pinch sensing element 52. The user may be prompted to perform a measurement, e.g. by an associated application installed on a mobile device.

The pinch sensing element 52 comprised within the band may by way of example, be any form of force or pressure sensor or a strain gauging device. Any other sensing element capable of providing an indication of applied force or pressure may also be used.

In accordance with any embodiment of the invention, the controller 34 may store or comprise or have access to a measurement schedule and be adapted to control generation of a sensory output at particular times in accordance with the schedule to prompt a user to perform a grip or pinching strength measurement. The sensory output may for example include a visual, auditory or haptic output.

By way of example, the clinical symptoms of rheumatoid arthritis (RA) show a circadian variation. An altered functioning of the hypothalamic-pituitary-adrenal axis (related to cortisol release) and of the pineal gland (related to melatonin release) seems to be important factors in the perpetuation of and circadian variation in symptoms of RA.

Consistently, human pro-inflammatory cytokine production exhibits a diurnal rhythmicity with peak levels during the night and early morning, at a time when plasma cortisol (anti-inflammatory) is lowest and melatonin (pro-inflammatory) is highest.

Sex hormones also seem to be involved in circadian rhythms of RA symptoms. Increased pain intensity and sleep disturbances are observed during the luteal phase in patients who have rheumatoid arthritis, when oestrogen (and progesterone) levels would be higher than in the follicular phase.

Furthermore research has been shown that disrupting the light-dark cycle of mice increased their susceptibility to inflammatory disease, again indicating that the production of a key immune cell is controlled by the body's circadian clock (Yu et al, (2013). Th17 cell differentiation is regulated by the circadian clock. Science, 342, 727-730).

It hence follows that the physical condition and the physical strength of rheumatic patients varies over the day and also varies between different days according to a predictable cycle. In particular, strength during the morning is less. This is hence reflected in grip strength, and patients with rheumatoid arthritis will exhibit predicable variations in grip strength throughout the day and day to day.

This variation has significant implications for patients, and in particular for making decisions about when to schedule certain activities of their daily life, about when to perform certain measurements in clinical trials, and about when to administer certain anti-rheumatic drugs, including corticosteroids and nonsteroidal anti-inflammatory drugs.

By collecting grip strength measurements at regular intervals, or at particular scheduled times throughout the day, an individualised variation of strength over the day can be determined, and this used to inform the above clinical and domestic factors.

In accordance with any embodiment of the device, the controller may be adapted to log generated signals indicative of a grip force, and optionally wherein the logging comprises storing the indications on a local memory, and/or comprises communicating the indications as output information to a remote data store. Thus, in accordance with these embodiments, the controller is adapted to perform recording or logging of the timing of a grip strength measurement.

The controller may in accordance with examples be further adapted to compare grip strength measurements taken across particular time windows from one day to the next.

To log the recorded grip strength indications, the controller may be adapted to store the indications on a local memory. Alternatively, the controller 34 may be adapted to communicate generated indications of grip strength, e.g. as data messages, to a remote server or a remote data store. Analysis of the data may be performed by a remote server receiving the communicated indications, for instance to compare the force indications of different time periods or different days.

In accordance with one or more embodiments, the grip measurement device may further comprise a user output element for generating a sensory output indicative of said signal indicative of the grip force. This may be a display element in examples. In examples, the grip measurement device may have further have means for providing wrist watch functionality, including for example a display at least for displaying a time, and a controller for implementing the wrist watch functionality.

As discussed above, various embodiments make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A wrist-worn grip measurement device (20) comprising:
a band (22) for fitting on a wrist (37);
a force sensing means (28, 42, 58) carried by the band for sensing a grip force applied to the device between at least two points (32a, 32b), said points being on surfaces exposed during wearing to permit application of the force by a user's alternate hand; and
a controller (34) adapted to generate a signal indicative of the grip force applied between said points based on an output of the force sensing means, and to generate an output based on said signal.

2. A wrist-worn grip measurement device (20) as claimed in claim 1, wherein the band (22) forms a closed loop shape when worn.

3. A wrist-worn grip measurement device (20) as claimed in any preceding claim, wherein the force sensing means (28, 42, 58) comprises a strain gauge means (28, 58) having a deformable element, and is adapted to provide a measure of a strain of the deformable element.

4. A wrist-worn grip measurement device (20) as claimed in any of claims 1-3, wherein the force sensing means (28, 42, 58) is integral to the band (22).

5. A wrist-worn grip measurement device (20) as claimed in any preceding claim, wherein
the band (22) includes a resilient circumferential portion (24) of greater relative resilience than a remaining circumferential portion (26),
the resilient circumferential portion being flexible inwardly of the band upon exertion of a gripping force by the user, and
the resilient circumferential portion incorporating strain gauge means (28), for measuring of a strain of the resilient portion upon said flexing.

6. A wrist-worn grip measurement device (20) as claimed in claim 5, the resilient circumferential portion (24) occupying less than half of a total circumference of the band (22),

7. A wrist-worn grip measurement device (20) as claimed in any one of claims 1 to 4, wherein:
the band (22) includes a set of rigid links; and
the force sensing means is provided between adjacent rigid links.

8. A wrist-worn grip measurement device (20) as claimed in any of claims 1-4, wherein
the force sensing means (28) comprises at least one pair of force sensor elements (42) carried by the band (22), and circumferentially spaced to permit gripping of the band between the force sensor elements to permit measurement of the user's grip.

9. A wrist-worn grip measurement device (20) as claimed in claim 8, wherein
the at least one pair of force sensor elements (42) are circumferentially separated by greater than one quarter of a circumference of the band (22); and/or
the at least one pair of force sensor elements are arranged diametrically opposite to one another.

10. A wrist-worn grip measurement device (20) as claimed in claim 8 or 9, wherein the band (22) carries a plurality of said pairs of force sensor elements (42), and optionally wherein said plurality of pairs are provided in the form of a sensor patch.

11. A wrist-worn grip measurement device (20) as claimed in any of claims 1-4, wherein the force sensing means comprises a pinch sensing element (52) which includes a layer element (56) having edges accessible to a user and being bendable by in-plane compression, and incorporating strain gauge means (58) to measure the bending and thereby provide a measure of a force of said in-plane compression.

12. A wrist-worn grip measurement device (20) as claimed in any of claims 1-4, wherein the wrist-worn device comprises a fastening element (27) to permit fastening of two ends of the band (22) around the user's wrist, and wherein the force sensing means (28) is comprised by the fastening means.

13. A wrist-worn grip measurement device (20) as claimed in any preceding claim, wherein the controller (34) is adapted to produce sensory signals in accordance with a stored measurement schedule to prompt a user to perform grip strength measurements at one or more scheduled times.

14. A wrist-worn grip measurement device (20) as claimed in any preceding claim, wherein the controller (34) is adapted to log generated signals indicative of a grip force, and optionally wherein the logging comprises storing the indications on a local memory, and/or comprises communicating the indications as output information to a remote data store.

15. A grip strength measurement method based on use of a wrist-worn grip measurement device (20) comprising
a band (22) for fitting on a wrist (37), and
a force sensing means (28, 42, 58) carried by the band for sensing a grip force applied to the device between at least two points, said points being on surfaces exposed during wearing to permit application of the force by a user's alternate hand,
the method comprising receiving one or more output signals from the force sensing means, generating a signal indicative of a grip force applied between said at least two points based on said one or more output signals, and generating an output based on said signal indicative of a grip force.
